(19) Europäisches Patentamt
European Patent Office
Office européen des brevets

(11) **EP 1 365 808 B1**

(12) **EUROPEAN PATENT SPECIFICATION**

(45) Date of publication and mention
of the grant of the patent:
**19.01.2011 Bulletin 2011/03**

(51) Int Cl.:
*A61P 35/00* (2006.01)    *A61K 45/06* (2006.01)
*A61K 31/495* (2006.01)

(21) Application number: **01980710.6**

(22) Date of filing: **06.11.2001**

(86) International application number:
**PCT/GB2001/004902**

(87) International publication number:
**WO 2002/036135 (10.05.2002 Gazette 2002/19)**

(54) **COMPOSITIONS FOR ANTITUMOUR TREATMENT CONTAINING ECTEINASCIDIN 743**

ANTITUMORZUSAMMENSETZUNGEN WELCHE ECTEINASCIDIN 743 ENTHALTEN

COMPOSITIONS CONTENANT D'ECTEINASCIDIN 743 POUR LE TRAITEMENT ANTI-TUMEUR

(84) Designated Contracting States:
**AT BE CH CY DE DK ES FI FR GB GR IE IT LI LU MC NL PT SE TR**
Designated Extension States:
**AL LT LV MK RO SI**

(30) Priority: **06.11.2000 US 246233 P**
**13.11.2000 US 248095 P**
**19.10.2001 US 345982 P**

(43) Date of publication of application:
**03.12.2003 Bulletin 2003/49**

(73) Proprietor: **PHARMA MAR, S.A.**
**28760 Tres Cantos,**
**Madrid (ES)**

(72) Inventors:
• **TAKAHASHI, Naoto**
**New York, NY 10021 (US)**

• **WEITMAN, Steve,**
**c/o Institute for Drug Development**
**San Antonio, TX 78245-3217 (US)**
• **D'INCALCI, Maurizio**
**Milan (IT)**
• **FAICLOTH, Glynn, Thomas,**
**c/o PharmaMar USA, Inc.**
**Cambridge, MA 02139-4616 (US)**
• **GIAVAZZI, Rafaella**
**I- Milan (IT)**
• **GESCHER, Andreas**
**Brand Hill,**
**Woodhouse Eaves LE12 8SS (GB)**

(74) Representative: **Ruffles, Graham Keith et al**
**Marks & Clerk LLP**
**62-68 Hills Road**
**Cambridge**
**CB2 1LA (GB)**

(56) References cited:
**US-A- 5 256 663**

**Description**

The present invention relates to effective antitumour treatments.

Ecteinascidin 743, ET743, is an anticancer agent derived from a marine source.

BACKGROUND OF THE INVENTION

The reader is referred to WO0069441 published 23 November 2000 for information on compositions and uses of ET743 for treating cancer which is to be considered pursuant to Art.54(3)EPC

SUMMARY OF THE INVENTION

In accordance with one aspect of this invention, we provide effective combination therapies based on ecteinascidin 743, using other drugs.

The object of the invention is defined in the claims.

PREFERRED EMBODIMENTS

The other drugs may form part of the same composition, or be provided as a separate composition for administration at the same time or a different time. The identity of the other drug is not particularly limited, and suitable candidates include:

   a) drugs with antimitotic effects, especially those which target cytoskeletal elements, including microtubule modulators such as taxane drugs (such as taxol, paclitaxel, taxotere, docetaxel), podophylotoxins or vinca alkaloids (vincristine, vinblastine);
   b) antimetabolite drugs such as 5-fluorouracil cytarabine, gemcitabine, purine analogues such as pentostatin, methotrexate);
   c) alkylating agents such as nitrogen mustards (such as cyclophosphamide or ifosphamide);
   d) drugs which target DNA such as the antracycline drugs adriamycin, doxorubicin, pharmorubicin or epirubicin;
   e) drugs which target topoisomerases such as etoposide;
   f) hormones and hormone agonists or antagonists such as estrogens, antiestrogens (tamoxifen and related compounds) and androgens, flutamide, leuprorelin, goserelin, cyprotrone or octreotide;
   g) drugs which target signal transduction in tumour cells including antibody derivatives such as herceptin;
   h) alkylating drugs such as platinum drugs (cis-platin, carbonplatin, oxaliplatin, paraplatin) or nitrosoureas;
   i) drugs potentially affecting metastasis of tumours such as matrix metalloproteinase inhibitors;
   j) gene therapy and antisense agents;
   k) antibody therapeutics;
   l) other bioactive compounds of marine origin, notably the didemnins such as aplidine;
   m) steroid analogues, in particular dexamethasone;
   n) anti-inflammatory drugs, in particular dexamethasone; and
   o) anti-emetic drugs, in particular dexamethasone.

As part of this patent specification, we include a series of examples and now refer to them. These examples demonstrate the increased effectiveness of ET-743 when used in combination with other drugs and are concerned with different combinations using ET-743.

Example 1 relates to effective combinations of ET-743 and doxorubicin for tumour growth inhibitions against marine and human sarcomas in athymic mice.

Example 2 shows ecteinascidin 743 (ET-743) and doxorubicin produce synergistic cytotoxic effects in soft tissue sarcoma lines HT-1080 and HS-18.

These two examples show more than additive effects of the combination of ET-743 with anthracyclines (in particular doxorubicin) which is more effective than either alone against human tumours (in these specific experiments sarcoma), which effects occur independent of sequence of administration. Such results show clear promise for treatment of patients.

Example 3 shows a synergistic cytoxic effect of ET-743 and cisplatin.

Example 4 provides a sequencing evaluation of ET-743 in combinations with chemotherapy agents against a panel of human tumour cell lines, in particular ET743 combinations with doxorubicin, taxol, SN-38, cisplatin, and gemcitabine.

These two show more than additive effects of the combination of ET-743 with platinum antitumour compounds, (in particular Cis-platin) with the nucleoside analogue gemcitabine, and with an inhibitor of topoisomerase II (SN38, which is the active agent produced from pro-drug CPT-11, a drug of the camptothecin group). Again these combinations are more effective than either drug alone against human tumours (in these specific experiments against a variety of tumour cells: ovarian, colon, lung, breast, bone sarcoma), which effects were dependent on sequence of exposure in some cases. Again there is promise for treatment of patients.

Interestingly, synergistic action was clearly not predictable: Example 4 indicates that in most combinations tested, no synergy was observed (in fact, antagonism was reported in some cases).

Example 5 relates to evaluation of combinations of Et-743 with doxorubicin or trimetrexate or paclitaxel

It shows more than additive effects of the combination of ET-743 with anthracyclines (in particular doxorubicin) which is more effective than either alone against human tumours (in these specific experiments sarcoma), which effects occur independent of sequence of administration. Such results show clear promise for treatment of patients.

Examples 6 to 8 reinforce and complement the previous examples, and especially show the synergy of ET-743 and doxorubicin and also ET-743 with cisplatin.

Example 9 demonstrates a different kind of effective ness of the combinations of this invention, where high-dose dexamethasone protects against the hepatotoxicity of ecteinascidin-743 (ET-743).

In summary, this invention therefore provides compositions for use in treatment, processes for preparing compositions and related embodiments.

The present invention also extends to the compounds of the invention for use in a method of treatment, and to the use of the compounds in the preparation of a composition for treatment of cancer.

Thus, the present invention can be used for treating any mammal, notably a human, affected by cancer which comprises administering to the affected individual a therapeutically effective amount of a compound of the invention, or a pharmaceutical composition thereof.

The present invention also relates to pharmaceutical preparations including a pharmaceutically acceptable carrier, which contain as active ingredient a compound or compounds of the invention, as well as the processes for their preparation.

Examples of pharmaceutical compositions include any solid (tablets, pills, capsules, granules, etc.) or liquid (solutions, suspensions or emulsions) with suitable composition or oral, topical or parenteral administration, and they may contain the pure compound or in combination with any carrier or other pharmacologically active compounds. These compositions may need to be sterile when administered parenterally.

Administration of the compounds or compositions of the present invention may be by any suitable method, such as intravenous infusion, oral preparations, intraperitoneal and intravenous administration. We prefer that infusion times of up to 24 hours are used, more preferably 2-12 hours, with 2-6 hours most preferred. Short infusion times which allow treatment to be carried out without an overnight stay in hospital are especially desirable. However, infusion may be 12 to 24 hours or even longer if required. Infusion may be carried out at suitable intervals of say 2 to 4 weeks. Pharmaceutical compositions containing compounds of the invention may be delivered by liposome or nanosphere encapsulation, in sustained release formulations or by other standard delivery means.

The correct dosage of the compounds will vary according to the particular formulation, the mode of application, and the particular *situs,* host and tumour being treated. Other factors like age, body weight, sex, diet, time of administration, rate of excretion, condition of the host, drug combinations, reaction sensitivities and severity of the disease shall be taken into account. Administration can be carried out continuously or periodically within the maximum tolerated dose.

The combinations of this invention can be used on refractory patients. The reader is referred to WO0069441 for information on dosing schemes for ET-743 and other information of use in the combination therapy of this invention.

EXAMPLES OF THE INVENTION

Example 1

Effective Combinations Of Et-743 And Doxorubicin For Tumor Growth Inhibitions Against Murine And Human Sarcomas In Athymic Mice

ET-743 has confirmed clinical activity in patients with soft and bone sarcoma refractory to previous chemotherapy including Doxorubicin (Dx) and Isosfamide. In view of the potential clinical value in combining ET-743 with Dx we have investigated this combination against the murine fibrosarcoma UV2237, its mdr-resistant subline UV2237/ADR and the human rabdomyosarcoma zenograft TE671. Both ET743 and Dx alone were effective against murine UV2237 fibrosarcoma whereas each was inactive or marginally active against both UV2237/ADR and TE671. However, the combination of ET743 and Dx was effective in all 3 models. The synergism was particularly marked in the human rabdomyosarcoma TE671 and appeared independent of drug sequence or combination.

After single i.v. treatments performed when the tumor TE671 was approximately of 100 mg tumor weight inhibition (TWI) and Log 10 Cell Kill (LCK) values were respectively 46% and 0.132 for ET-743 (0.1 mg/kg) alone, 50% and 0.33 for Dx (10 mg/kg) alone, 77% and 0.924 for ET-743 (0.1 mg/kg) and Dx (10 mg/kg) given simultaneously, 82% and 1.12 for the combination of ET-743 (0.1 mg/kg) given 1 hour before Dx (10 mg/kg) and 75% and 0.85 for the combination of ET-743 (0.1 mg/kg) given 1 h after Dx (10 mg/kg).

These data suggest that the combination of ET-743 and Dx can also be effective in tumors that are not sensitive or marginally sensitive to these drugs given alone, thus providing a strong rationale for clinical investigations using this combination.

Example 2

Ecteinascidin 743 (et-743) and Doxorubicin Produce Synergistic Cytotoxic Effects in Soft Tissue Sarcoma Lines HT-1080 and HS-18.

Two sarcoma cell lines, HT 1080, a fibrosarcoma cell line sensitive to ET-743 IC$_{50}$ = 10pm) and HS-18, a liposarcoma cell line, less sensitive to ET-743 (IC$_{50}$ = 270pm) were evaluated for toxicity to ET-743 in combination with either doxorubicin, trimetrexate or paclitazel. When ET-743 was used in combination with each of these drugs at a constant molar ration, and analysed by the method of Chou and Talalay, synergistic effects were obtained (72 hr incubation) with the ET-743-doxorubicin combination, but not with the combination of ET-743 with trimetrexate or paclitaxel When cells were exposed to ET-743 for 72 hr, and either doxorubicin, trimetrexate or taxol for the last 48 hrs of incubation, synergistic effects were also obtained with doxorubicin against both sarcoma cell lines. Of interest, the sequence paclitaxel followed by ET-743 was more effective than the opposite sequence. These results encourage clinical trials of doxorubicin in combination with ET-743 to treat patients with soft tissue sarcoma, as both of these drugs have shown activity against this disease.

Example 3

Synergistic Cytotoxic Effect Of Et-743 And Cisplatin

Ecteinascidin 743 (ET-743) has shown striking antitumor activity in several preclinical systems and promising clinical activity. ET-743 binds N2 guanines in the minor groove and affects the regulation of transcription (Minuzzo et aL, PNAS, Vol. 97,6780-84, 2000).

Previous studies have indicated that mismatch repair (MMR) deficient cells are equally sensitive to ET-743 as proficient cells. NER deficient cells very sensitive to cisplatin are 6-8 times less sensitive to ET-743. On the basis of the different mechanisms involved in the repair of ET-743 and cisplatin and because of the potential clinical interest in this combination we have performed studies to evaluate the cytotoxic effects of ET-743 and cisplatin in several human tumor cell lines. Human ovarian cancer Igrove-1 cell line, a subline resistant to ET-743 (IG/PSC/ET), human colon cancer HCT 116, (MMR deficient) and HCT11-ch3 (MMR proficient) cell lines were used in this study.

The cells were treated for 1 or 24 h with different concentrations of ET-743 or cisDDP, alone or in combinations, and the cytotoxicity was evaluated by using a colorimetric assay after sulforodhamine B staining. In all the cell lines a synergistic effect was observed both with 1 h or 24 h exposure. Interestingly in HCT116 resistant to cisDDP ET-743 was apparently able to reverse sensitivity even at concentrations of ET-743 which alone were marginally effective. Taken together the data provide a rational for undertaking clinical studies combining ET-743 with cisDDP.

Example 4

Et743 Combinations With Doxorubicin, Taxol, Sn-38, Cisplatin, And Gemcitabine

ET-743 was evaluated in combination with doxorubicin, taxol, SN-38, cisplatin, and gemcitabine against a panel of human tumor cell lines. These studies were designed to determine the type of drug-drug interaction between ET-743 and standard chemotherapy agents and the influence of sequence of exposure on antitumor activity. Multiple combinations of ET-743 with standard cytotoxic agents were used with a model-free design (Laska, et al. Biometrics 50:834, 1994) to describe the type of drug-drug interaction. These studies suggest that regardless of exposure, an additive pattern of drug-drug interaction is most typically observed.

A synergistic drug-drug interaction was observed when ET-743 was combined against non-small cell lung (pre-exposure to SN-38), osteosarcoma (pre-exposure with ET-743 followed by cisplatin), breast (pre-exposure to ET-743 followed by gemcitabine), colon (pre-exposure with ET-743 followed by SN-38 and concurrent exposure with SN-38) tumor cell lines. An additive/synergistic (pre-exposure to ET-743 followed by SN-38 against NSCL; pre-exposure to SN-38 against colon and NSCL; concurrent exposure with cisplatin against osteosarcoma, and with SN-38 against NSCL lines) pattern of drug-drug interaction was observed. Evidence of antagonism was noted when taxol was utilized concurrently against two NSCL lines, and doxorubicin against a rhabomyosarcoma cell line.

These studies suggest that ET-743 which is in Phase II clinical trials, could be combined with several cytotoxic agents against a broad-range of tumor types.

Material And Methods

Cell culture:

Human breast (MDA-435, MDA-231, T-470), non-small cell lung (NCI-H522, NC1-H226, NCI-H23), colon (HCT-116, HT-29, Colo-320), osteosarcoma (HOS, U-2, OS, SaOS-2), rhabdomyosarcoma (RH1, RH30, RD) tumor cell lines were grown in RPMI-1640 supplemented with 10% fetal bovine serum and 2mM L-glutamine. All stock cultures were maintained in 75 cm$^{-2}$ flasks at 37°C in humidified incubators with a 5% $CO_2$-95% air atmosphere.

$IC_{50}$ Analysis:

A pre-determined number of exponentially growing tumor cells were inoculated in 96-well tissue culture plates and allowed to stabilize for 24 hours. Afterwards, a drug plate consisting of serial diluted concentrations of ET-743 or standard chemotherapy agents was added to the cells. Cells were incubated as a 24-hour exposure for three days followed by the addition of MTT for 4 hours. Resultant formazan crystals were then solubilized with acid/ alcohol, with absorbance (570 nm-test/630 nm-reference) determined using a microplate reader. Results were expressed as percent tumor cell kill compared to media controls.

Combination Studies:

For the combination studies, the concentration (expressed as a percent of the individual agent's $IC_{50}$) schema used to characterize the type of interaction is shown below:

| Drug Concentration (Expressed as a percent of the $IC_{50}$) | |
| --- | --- |
| ET-743 | Standard agents |
| 100 | 0 |
| 75 | 25 |
| 60 | 40 |
| 50 | 50 |
| 40 | 60 |
| 25 | 75 |
| 0 | 100 |
| 0 | 0 |

Statistical Analysis of Combination Studies:

Statistical comparisons are made with each test combination (75:25-ET-743/standard agents) and the endpoints (100:0-ET-743 and 0:100-standard agents). A statistically significant observation requires that a difference exists between the combination (ET-743 and standard agents) absorbance value and both endpoint values (ET-743 and standard agents alone). If the majority of ($\geq$3 of 5) the values are statistically above or below the line then antagonism or synergy is described, respectively. Otherwise the pattern is more consistent with an additive interaction. Interpretation is very difficult if there is considerable slope to the line connecting the endpoints. If the slope of the $IC_{50}$ curves for the individual agents are identical (unlikely) then you can, at times, determine the type of interaction.

| Sequencing Combination of ET-743 with Chemotherapy Agents | | |
| --- | --- | --- |
| Tumor Type/Cell Line | Exposure Conditions/Agents | Drug-Drug Interactions Observed |
| Osteosarcoma | | |
| NOS | 24 hour ET-743 followed by 24 hour exposure to cisplatin | Synergistic |
| | 24 hour cisplatin followed by 24 hour exposure to ET-743 | Additive |
| | 24 hour concurrent ET-743/cisplatin exposure | Additive |
| U2-OS | 24 hour ET-743 followed by 24 hour exposure to cisplatin | Additive |
| | 24 hour cisplatin followed by 24 hour exposure to ET-743 | Additive |
| | 24 hour concurrent ET-743/cisplatin exposure | Additive |
| Sa06 | 24 hour ET-743 followed by 24 hour exposure to cisplatin | Additive |
| | 24 hour cisplatin followed by 24 hour exposure to ET-743 | Additive |
| | 24 hour concurrent ET-743/cisplatin exposure | Additive/Synergistic |
| Non-Small Cell Lung | | |
| | 24 hour ET-743 followed by 24 hour exposure to taxol | Additive |
| NCB-H226 | 24 hour taxol followed by 24 hour exposure to ET-734 | Additive |
| | 24 hour concurrent ET-743/taxol exposure | Antagonistic |
| | 24 hour ET-743 followed by 24 hour exposure to SN38 | Additive/Synergistic |
| | 24 hour SN-38 followed by 24 hour exposure to ET-743 | Adaitive/Synergistic |
| | 24 hour concurrent ET-743/SN-38 exposure | Additive |
| NCB-N522 | 24 hour ET-743 followed by 24 hour exposure to taxol | Additive |
| | 24 hour taxol followed by 24 hour exposure to ET-734 | Additive |
| | 24 hour concurrent ET-743/taxol exposure | Antagonistic |
| | 24 hour ET-743 followed by 24 hour exposure to SN38 | Additive/Synergistic |
| | 24 hour SN-38 followed by 24 hour exposure to ET-743 | Additive/Spnergistic |
| | 24 hour concurrent ET-743/SN-38 exposure | Additive |
| NCB-N23 | 24 hour ET-743 followed by 24 hour exposure to taxol | Additive/Antagonistic |
| | 24 hour taxol followed by 24 hour exposure to ET-734 | Additive |
| | 24 hour concurrent ET-743/taxol exposure | Antagonistic |
| | 24 hour ET-743 followed by 24 hour exposure to SN38 | Additive |
| | 24 hour SN-38 followed by 24 hour exposure to ET-743 | Synergistic |
| | 24 hour concurrent ET-743/SN-38 exposure | Additive/Synergistic |
| Breast | | |
| MDA-435 | 24 hour ET-743 followed by 24 hour exposure to gerncitabine | Additive |
| | 24 hour gerncitabine followed by 24 hour exposure to ET-743 | Additive |
| | 24 hour concurrent ET-473/germcitabine | Additive |
| MDA-231 | 24 hour ET-743 followed by 24 hour exposure to gerncitabine | Additive |

(continued)

| Breast | | |
|---|---|---|
| | 24 hour gerncitabine followed by 24 hour exposure to ET-743 | Additive |
| | 24 hour concurrent ET-473/germcitabine | Additive |
| T47-8 | 24 hour ET-743 followed by 24 hour exposure to gerncitabine | Additive |
| | 24 hour gerncitabine followed by 24 hour exposure to ET-743 | Additive |
| | 24 hour concurrent ET-473/germcitabine | Additive |
| Colon | | |
| MCT-116 | 24 hour ET-743 followed by 24 hour exposure to SN-38 | Synergistic |
| | 24 hour ET-743 followed by 24 hour exposure to SN-38 | Additive |
| | 24 hour concurrent ET-743/SN exposure | Additive |
| NT-29 | 24 hour ET-743 followed by 24 hour exposure to SN-38 | Additive |
| | 24 hour ET-743 followed by 24 hour exposure to SN-38 | Additive |
| | 24 hour concurrent ET-743/SN exposure | Additive |
| Colo-320 | 24 hour ET-743 followed by 24 hour exposure to SN-38 | Additive |
| | 24 hour ET-743 followed by 24 hour exposure to SN-38 | Additive/Synergistic |
| | 24 hour concurrent ET-743/SN exposure | Synergistic |
| rhabdoroyo-sarcoma | | |
| RN1 | 24 hour ET-743 followed by 24 hour exposure doxorubicin | Additive |
| | 24 hour doxorubicin followed by 24 hour exposure to ET-743 | Additive |
| | 24 hour concurrent ET-743/doxorubicin exposure | Antagonistic |
| RD | 24 hour ET-743 followed by 24 hour exposure doxorubicin | Additive |
| | 24 hour doxorubicin followed by 24 hour exposure to ET-743 | Additive |
| | 24 hour concurrent ET-743/doxorubicin exposure | Additive/Antagonistic |
| RN30 | 24 hour ET-743 followed by 24 hour exposure doxorubicin | Additive |
| | 24 hour doxorubicin followed by 24 hour exposure to ET-743 | Additive |
| | 24 hour concurrent ET-743/doxorubicin exposure | Antagonistic |

Conclusions-Summary

These studies suggest that regardless of sequence of exposure between ET-743 and standard chemotherapy agents, an additive pattern of drug-drug interaction is most typically observed.

Evidence of synergy was observed when NC1-H522 and NC1-H23 NSCL lines were pre-exposed to SN-38, pre-exposure to ET-743 with cisplatin against HOS osteosarcoma, T-470 breast cell line with gerncitabine, SN-38 against HCT-116 colon, and concurrent exposure with SN-38 against Colo-320 colon tumor cell line.

Evidence of antagonism was observed when taxol was utilized concurrently against the NC1-H226 and NC1-H23 NSCL cell line and doxorubicin against the RHI rhabdomyosarcoma tumor cell line.

Example 5

Interaction Between Et-743 And Other Antineoplastic Agents

Although ET-743 is presently in clinical trials from human cancers, the mechanisms of antitumor activity of ET-743 have not been completely elucidated. The aim of this study was to assess the nature of the interaction between ET-743 and other antineoplastic agents (doxorubicin; DXR, trimetrexate; TMTX and Paclitaxel; Taxon) using the combination index (CI) method of Chou and Talalay. To better understand how ET-743 might be used clinically, the present study used SRB assays to examine the cytotoxicity resulting from combining ET-743 with three other antineoplastic agents in the different administration schedules in two soft tissue sarcoma cell lines, HT-1080 and HS-18, in vitro. DXR was the only agent that resulted in sequence-independent synergy when combined with ET-743. Concurrent exposure of ET-743 with DXR resulted in synergistic interactions in both cells lines.

The CIs (mean) with the schedule were 0.86, 0.83, 0.84 and 0.85 at 50, 75, 90 and 95% cell kill, respectively, in HT-1080 cells and 0.89, 0.74, 0.64 and 0.60 at 50, 75, 90 and 95% cell kill, respectively, in HS-18 cells. Sequencing with ET-743 for 24 h prior to DXR was the most effective regimen against both cell lines; it resulted in consistently low CI of up to the about 90% cell kill level for both cell lines. Exposure to Taxol prior to ET-743 was also an effective regimen. These results suggest that the combination of ET-743 and DXR should be explored further in clinical trials in the treatment of soft tissue sarcoma.

Materials And Methods

Chemicals

ET-743 was provided by Pharma-Mar S.A (Tres Cantos, Madrid, Spain), and was prepared as a 2 mM stock solution in dimethyl sulfoxide. Paclitaxel and DXR were obtained from Sigma chemical Co. (St. Louis, MO). TMTX was supplied by Warner-Lambert (Parke-Davis, Ann Arbor, Mich).

Cell Culture

Soft tissue sarcoma cell lines, HT-1080 and HS-18 were maintained as monolayer cultures in RP<I-1640 containing 10% fetal bovine serum.

SRB Cytotoxicity Assay

Cytotoxicity to drugs was determined by SRB cytotoxicity assay carried out in 96-well microtiter plates as described. Cells were plated in duplicate wells (5000 cells/well) and exposed to drugs at different concentrations. Cells were fixed with 50% TCA solution for 1 h and 0.4% SRB (Sigma) was added to each well. After a 30 min incubation, the plate were washed with 1% acetic acid and read at 570 nm on a Biowhitaker microplate reader 2001. The wells with cells containing no drugs and with medium plus drugs but no cells were used as positive and negative controls, respectively.

Concurrent Exposure to ET-743 and DXR, TMTX or Paclitaxel

Cells were seeded into 96-well plates, as described previously. Cells were treated with seven different concentrations of the single drugs or combinations mixture at 1:100 (ET-743 : the other drugs) molar ratio. After 72 h exposure, growth inhibition was measured using the SRB assay.

Sequential Exposure to ET-743 and DXR, TMTX or Paclitaxel

Using the same experimental setup described above, we exposed cells to three different concentrations of drugs which represents the $IC_{25}$, $IC_{50}$, $IC_{75}$ of ET-743, DXR, TMTX and paclitaxel, respectively. After 24 hours pre-treatment with ET743 or the combination drug, the second drugs were added to the respective wells for 48 h. Growth inhibition was determined using the SRB assay.)

Cell Cycle Analysis

Exponentially growth cells were treated with or without drugs for several hours. Cells were then collected and fixed with ice-cold 70% methanol DNA was stained with propidium iodide as described previously. Ten thousand stained cells were analyze on a Becton Dickinson fluorescence-activated cell sorter (FACS).

Determination of Synergism and Antagonism and Construction of Isobolograms

The CI was calculated by the Chou-Talalay equation, which takes into account both potency (Dm or $IC_{50}$) and the shape of the dose effect curve (the m value). The general equation for the classic isobologram (CI = 1) is given by:

$$CI = (D)_1 / (Dx)_1 + (D)_2 / (Dx)_2 \qquad (A)$$

where $(Dx)_1$ and $(Dx)_2$ in the denominators are the doses (or concentrations) for $D_1$ (ET-743) and $D_2$ (another drug) alone that give X % inhibition, whereas $(D)_1$ and $(D)_2$ in the numerators are doses of ET-743 and another drug in combination also inhibited X % (ie isoeffective). CI < 1, CI = 1, CI > 1 indicated synergism, additive effect and antagonism, respectively.

The $(Dx)_1$ or $(Dx)_2$ can be readily calculated from the median-effect equation of Chou and Chou et al:

$$Dx = Dm [fa / (1 - fa)]^{1/m} \qquad (B)$$

where Dm is the median-effect dose that is obtained from the anti-log of the X-intercept of the median-effect plot, X-log (D) versus Y - log [fa / (1- fa)] or $Dm = 10^{-(Y\text{-intercept}) / m}$, and m is the slope of median-effect plot Computer software of Chou and Chou allows automated calculation of m, Dm, Dx, and CI values. From $(Dm)_1$, $(Dx)_2$, and D1 + D2, it becomes easy to constract isobolograms automatically based on Eq. A.

For conservative mutually nonexclusive isobolograms of two agents, a third term,

$$(D1) (D2) / (DX)_1 (DX)_2 \qquad (C)$$

is added to Eq. A.

For simplicity, the third term is usually omitted, and thus the mutually exclusive assumption or classic isobologram is indicated. In Result 2 and 3, the CI values obtained from the classic (mutually exclusive) calculation are given.

Result 1

| Cytotoxicity of four drugs on HT-1080 and S18 | | | |
|---|---|---|---|
| | $IC_{50}$ for human soft tissue sarcoma cells | | |
| | | HT-1080 | HS-18 |
| ET-743 . | (nM) | 0.01 | 0.27 |
| DXR | (nM) | 25 | 225 |
| TMTX | (nM) | 6 | 70000 |
| Paclitaxel | (nM) | 1:3 | 10 |

This table showed that both HT-1080 and S18 cell lines were more sensitive to ET-743 than other antineoplasic agents.

| Effect of each agent on cell cycle distribution against HS-18 cells 24 h and 72 h after treatment with approximate $IC_{50}$ dose | | | | | |
|---|---|---|---|---|---|
| Drugs | Dose | HR | %G1 | %S Phase | %G2-M |
| Control | | | 76.3 | 11.2 | 12.5 |
| ET-743 | 270 pM | 24 | 32.4 | 47.6 | 20.0 |
| | | 72 | 86.7 | 8.4 | 4.9 |
| DXR | 225 nM | 24 | 10.1 | 64.9 | 25.0 |
| | | 72 | 1.3 | 63.8 | 34.9 |
| TMTX | 70 uM | 24 | 44.2 | 53.8 | 1.9 |
| | | 72 | 35.5 | 57.6 | 7.0 |
| Paclitaxel | 10 nM | 24 | 32.8 | 52.5 | 15.5 |
| | | 72 | 23.5 | 58.7 | 26.2 |

| Effect of each agent on cell cycle distribution against HT-1080 cells 24 h and 72 h after treatment with approximate $IC_{50}$ dose | | | | | |
|---|---|---|---|---|---|
| Drugs | Dose | Hr | %G1 | %S phase | %G2-M |
| Control | | | 47.5 | 35.8 | 16.7 |
| ET-743 | 10 pM | 24 | 42.6 | 36.1 | 21.3 |
| | | 72 | 83.1 | 10.2 | 6.7 |
| DXR | 25 nM | 24 | 36.1 | 17.5 | 46.4 |
| | | 72 | 46.2 | 5.3 | 48.5 |
| TMTX | 6 nM | 24 | 31.9 | 56.8 | 11.3 |
| | | 72 | 32.0 | 53.7 | 14.4 |
| Paclitaxel | 1.3 nM | 24 | 45.4 | 37.3 | 17.3 |
| | | 72 | 86.0 | 9.0 | 5.0 |

Result 2 shows the CI for HT-1080 and HS-18 cells, respectively, which were simultaneously exposed to ET-743 and one of antineoplastic drugs, such as DXR, TMTX or paclitaxel, at 1 to 100 molar ratio combination mixture. When cells were treated with ET-743 and DXR, the CI values were all below 1, indicating synergism effect in both cell lines. The CI (mean) with this schedule were 0.86, 0.83, 0.84 and 0.85 at 50, 75, 90 and 95% cell kill, respectively, in HT-1080 cells and 0.89, 0.74, 0.64 and 0.60 at 50, 75, 90 and 95% cell kill, respectively, in HS-18 cells. This result showed that concurrent treatment of ET-743 and DXR produced synergistic sytotoxic effect. In contrast, when cells were treated with ET-0743 and TMTX or paclitaxel, antagonism cytotoxic effect was observed

The CI plot was obtained from both cell lines which were initially exposed to ET-743 for 24 h, followed by DXR for 48 h. In both cells lines, ET-743 followed by DXR treatment showed synergistic cytotoxic effect, the CI value of HT-1080 at 80% cell kill level was $0.64 \pm 0.12$ and that of HS-18 at 88% cell kill level was $0.24 \pm 0.06$. In contrast, DXR followed by ET-743 treatment (Result 3a, lower figure) demonstrated the good CI value at first sight however, the CI value of HT-1080 at 80% cell kill level was $1.00 \pm 0.03$, indicating that the effect of the two agents were additive, in addition, the CI at highest fraction killed was worse than that at middle fraction killed in both cells.

When cells were exposed to ET-743 followed by TMTX, the CI values of HT-1080 showed nearly one or over one, indicating that the effect of the two agents are antagonism or additive. In contrast, those of HS-18 were all under 0.6, demonstrating that these two drugs have synergy effect When cells were treated with TMTX followed by ET-743, additive effect was observed in both HT-1080 and HS-18 cell lines.

Paclitaxel followed by ET-743 treatment produced synergistic cytotocix effect. When cells were exposed to paclitaxel followed by ET-743, the CI value of HT-1080 at 89% cell kill level was $0.92 \pm 0.06$ and that of HS-18 at 78% cell kill level was $0.38 \pm 0.13$.

Summary

ET-743 was highly active against human soft tissue sarcoma cells, especially against the malignant fibrosarcoma cell line HT-1080.

DXR resulted in sequence-independent synergy when combined with ET-743, however, sequencing with ET-743 followed by DXR was more effective against both cell lines.

Exposure to paclitaxel followed by ET-743 was also an effective regimen against human soft tissue sarcoma cells, while concomitant exposure was antagonistic.

Example 6

In vivo combinations of chemotherapeutic agents with Ecteinascidin 743 (Et743) against solid tumors.

Several unique mechanisms of action have been described for Et743 including binding to the minor groove of DNA, alkylation of the N2 of guanine, transcriptional inhibition of MDR1 gene (jin et al., PNAS 97, 6775, 2000; Minuzzo et aL, PNAS 97, 6780, 2000) and counteracting the activation of nuclear receptor SXR (Synold et aL, Nature Med 7, 584, 2001). As a single agent, Et743 inhibits *in vivo* tumor growth achieving complete remissions (CR) against several human tumor strains (Hendriks et al., Ann Oncol 10,1233, 1999) including melanoma (MEXF 989), NSCL (LXFL 529), ovary (HOC 22) and breast carcinoma (mix-1). The effectiveness of Et743 in combination with drugs that work by alternate mechanisms may provide opportunities to reduce the toxicities of either drug or to potentiate the effectiveness of a drug in resistant or relapsed cancers.

For this evaluation several agents including doxorubicin (DOY, 8 mg/Kg), cisplatin (DDP; 12 mg/Kg) and vinblastine (VINB; 6 mg/Kg) were administered before/after Et743 (0.2 mg/Kg) with 1 hour pretreatment, qdx5, in one or more of the following tumors: chondrosarcoma (CSHA), osteosarcoma (OSA-FH), fibrosarcoma (SW684), ovary (MRI-H-1834), NSCL (LX-1) and renal (MRI-H-121) with activity defined as < 50% T/C. In the hollow fiber (HF) model, the sequence of DOX, 1 hr pre-Et743 was consistently more effective than Et743 alone in chondrosarcoma (6% vs. 10%), fibrosarcoma (33% vs. 48%) and osteosarcoma (20% vs. 34%). Osteosarcoma xenografts produced similar results of 17% vs. 43%. HF studies with DPP showed that Et743 pre-DDP was more effective than Et743 alone in ovary (28% vs. 100%) and chondrosarcoma (15% vs. 19%) and equivalent activities in osteosarcoma (36% T/C). Xenograft data confirms the sequence of Et743 pre-DDP as more effective than Et743 alone (35% vs. 66%). The one exception was in NSCL were Et743 alone was not active (62% T/C) but DPP followed by Et743 produced CR (<1% T/C). In renal xenografts Et743 alone was very active (22% T/C) but Et743 followed by VINB also produced CR (<1% T/C). Separate studies are underway with other standard agents in breast, renal, melanoma and gastric tumor xenografts.

Example 7

Preclinical activity and biodistribution of Ecteinascidin 743 (ET-743) and Doxorubicin (DOX) combinations in human rhabdomyosarcoma.

ET-743 is the first of a new class of antitumor agents that exhibits anti-tumor activity. ET-743 has shown activity in patients with sarcoma refractory to DOX and ifosfamide. In view of its potential as an effective drug, we investigated (1) the preclinical anti-tumor activity of ET-743/DOX combination against the human rhabdomyosarcoma TE 671 and (2) possible interactions between the drugs and their biodistribution in nude mice and tumor xenografts.

*In vitro:* The effect of each drug or combination after 1 hr exposures was evaluated by clonogenic assay. ET-743 or DOX alone showed anti-tumor activity against TE 671 cells. The combination according to isobologram analysis and Combination Index, was at least additive in several tumor cell lines including TE 671.

*In vivo:* Single iv treatments (ET-743, 0.1mg/Kg; DOX, 10mg/Kg) were administered in nude mice when xenograft tumors weighed approximately 100 mg. Tumor weight inhibition/Log10 Cell Kill values were 46%/0.132 for ET alone, 50%/0.33 for DOX alone, 77%/0.924 for ET-743 and DOX given simultaneously, 82%/1.12 for the combination of ET-743 given 1h before DOX, and 75%/0.85 when ET-743 was given 1h after DOX. A synergistic effect has also been observed against the murine fibrosarcoma UV2237 and against its multidrug resistant subline UV2237/ADR

These data show a synergistic effect of ET-743/DOX and appears to be independent of drug sequence or combination in the scenarios studied thus far. Neither the plasma nor the tumor concentrations of DOX are significantly different when DOX was given alone or in combination with ET-743. The pharmacokinetic (PK) evaluation of ET-743 given alone or in combination with DOX is underway. The combination of ET-743 and DOX appears additive in vitro yet synergistic in vivo in rhabdomyosarcoma TE 671. The PK profile of DOX is not influenced by concomitant treatment with ET-743. These data provide a rationale for using this combination in early clinical trials.

Example 8

ET-743 and cisplatin (DDP) show in vitro and in vivo synergy against human sarcoma and ovarian carcinoma cell lines.

We show here that ET-743 enhances the activity of DDP both in vitro and in vivo. In several cancer cell lines including human intestinal carcinoma (HCT116), ovarian carcinoma (Igrov-1, A2780), their resistant sublines (Igrov-1/PSC-ET and 1A9, respectively), and rabdomyosarcoma (TE671), lower concentrations of ET-743 used as a single agent could potentiate DDP activity by at least 2-fold Concentrations corresponding to IC30/IC50 of ET-743 resulted in either additive or synergistic effects. These results have led to in vivo studies using xenograft models to study effective drug combinations with ET-743.

In sc transplanted TE671, partially sensitive to either ET-743 and DDP, the combination of the two drugs produced an antitumor effect much greater than that achieved with either drug used at their respective MTD levels. The ovarian 1 A 9 tumor that is normally resistant to both ET-743 and DDP as sinsgle agents, in combination produced a tumor growth inhibition greater than 50%. Orthotopically transplanted human ovarian carcinoma $HOC_8$, producing tumor nodules in the peritoneal cavity with ascitis, which is resistant to ET-743 and partially sensitive to DDP, in combination resulted in a dramatic increase in survival even at the dose of ET-743 of 0.05 mg/Kg (¼ MTD) and did not cause any significant toxicity. An ET-743 dose of 0.15 mg/Kg markedly increased survival, but there was also an increase in toxicity as indicated by a weight loss, that was significantly higher than that observed after treatment with each drug.

These findings offer a strong rationale to design clinical trials using the combination of ET-743 and DDP in sarcomas and ovarian cancers. *In vitro* and *in vivo* studies are in progress to elucidate the mechanisms underlining the synergism between ET-743 and DDP in these cancer types.

Example 9

High-Dose Dexamethasone (dex) Protects Against the Hepatotoxicity of Ecteinascidin-743 (ET-743) in theRat

ET-743, an agent derived from a marine tunicate, is currently in phase II clinical trial. It has shown clinical activity against sarcomas, and preliminary data suggests activity against breast and ovarian carcinoma. However, hepatotoxicity characterized by reversible transaminitis occurs in most treated patients and cholestasis in a minority. In the most sensitive animal species, the rat, toxicity of ET-743 is characterized by hepatic necrosis and bile duct inflammation. In the light of the antiinflammatory activity of dex, we investigated its effect on liver damage induced by ET-743 in the rat. Female Wistar rats received a single iv dose of ET-743 (40 µg/kg). Some rats were pretreated with a single oral dose of dex either at 1, 5, 10 or 20 mg/kg 24 h prior to ET-743 treatment. Liver pathology and plasma concentrations of alkaline phophatase (ALP), aspartate aminotransferase (GOT) and total bilirubin (TB) were assessed up to 3 days *post* ET-743 administration. Conventional histological sections of the livers were examined by light microscopy.

At 2 days *post* ET-743 treatment, livers from rats that received ET-743 alone showed bile duct inflammation, striking degenerative changes in biliary epithelial cells and zones of hepatic necrosis. Plasma levels of ALP and GOT were significantly elevated after 2 days. Cholestasis was reflected by a dramatic increase in plasma TB concentrations, which commenced on day 2 after ET-743. ET-743-induced histopathological changes and elevation of plasma ALP, GOT and TB were totally abrogated in rats pre-treated with 10 or 20 mg/kg dex.

Whilst dex at 1 mg/kg showed little protection, 5 mg/kg was moderately protective. Plasma levels of ET-743 in rats which received dex (50 mg/kg) daily for 3 days prior to ET-743 were not decreased compared to those in rats on ET-743 alone. Furthermore, the activity of ET-743 against B16 melanoma implanted into mice was not impeded by dexamethasone. These findings suggest that the addition of high-dose dexamethasone to the ET-743 regimen may ameliorate its hepatotoxicity in cancer patients.

**Claims**

1. The use of a synergistic combination of ET-743 and another drug selected from an anthracycline drug, a platinum drug, a topoisomerase-targeting drug, a taxane drug, an antimetabolite drug, or an antimitotic drug in the manufacture of a medicament for the treatment of a tumor.

2. The use according to claim 1, wherein the combination therapy employs ET-743 and doxorubicin.

3. The use according to claim 1, wherein the combination therapy employs ET-743 and cisplatin.

4. The use according to claim 1, wherein the combination therapy employs ET-743 and paclitaxel.

**5.** The use according to claim 1, wherein the combination therapy employs ET-743 and gemcitabine.

**6.** The use according to claim 1, wherein the combination therapy employs ET-743 and SN-38.

**7.** The use according to claim 1, wherein the combination therapy employs ET-743 and trimetrexate.

**8.** The use according to claim 1, wherein the combination therapy employs ET-743 and vinblastine.

**9.** The use according to any of claims 2, 3, 4 or 7 for the treatment of sarcoma.

**10.** The use according to claim 3 or 6 for the treatment of colon cancer.

**11.** The use according to claim 3 for the treatment of intestinal carcinoma.

**12.** The use according to claim 3 or 6 for the treatment of lung cancer.

**13.** The use according to claim 5 for the treatment of breast cancer.

**14.** The use according to claim 8 for the treatment of renal cancer.

**15.** The use according to any preceding claim, wherein ET-743 and the other drug are provided as a single medicament.

**16.** The use according to any of claims. 1 to 14, wherein ET-743 and the other drug are provided as separate medicaments.

**17.** The use according to claim 16, wherein the separate medicament containing ET-743 is for administration at the same time as the medicament containing the other drug.

**18.** The use according to claim 16, wherein the separate medicament containing ET-743 is for administration at a different time as the medicament containing the other drug.

**19.** The use according to any preceding claim, wherein the other drug or the combination is delivered by liposome or nanosphere encapsulation.

**20.** The use according to any preceding claim, wherein the combination therapy further employs dexamethasone.

**21.** A synergistic combination of ET-743 and another drug selected from an anthracycline drug, a platinum drug, a topoisomerase-targeting drug, a taxane drug, an antimetabolite drug, or an antimitotic drug for use in the treatment of a tumour as described in any preceding claim.

**Patentansprüche**

**1.** Verwendung einer synergistischen Kombination von ET-743 und einem anderen Wirkstoff, der unter einem Anthracyclin- Wirkstoff, einem Platin-Wirkstoff, einem Wirkstoff mit Topoisomerase als Target, einem Taxan-Wirkstoff, einem Antimetaboliten-Wizkstoff oder einem antimitotischen Wirkstoff ausgewählt ist, bei der Herstellung eines Medikaments für die Behandlung eines Tumors.

**2.** Verwendung nach Anspruch 1, wobei die Kombinationstherapie ET-743 und Doxorubicin verwendet.

**3.** Verwendung nach Anspruch 1, wobei die Kombinationsthezapie ET-743 und Cisplatin verwendet.

**4.** Verwendung nach Anspruch 1, wobei die Kombinationstherapie ET-743 und Paclitaxel verwendet.

**5.** Verwendung nach Anspruch 1, wobei die Kombinationstherapie ET-743 und Gemcitabin verwendet.

**6.** Verwendung nach Anspruch 1, wobei die Kombinationstherapie ET-743 und SN-38 verwendet.

**7.** Verwendung nach Anspruch 1, wobei die Kombinationstherapie ET-743 und Trimetrexat verwendet.

**8.** Verwendung nach Anspruch 1, wobei die Kombinationstherapie ET-743 und Vinblastin verwendet.

**9.** Verwendung nach einem der Ansprüche 2, 3, 4 oder 7 für die Behandlung von Sarkom.

**10.** Verwendung nach Anspruch 3 oder 6 für die Behandlung von Dickdarmkrebs.

**11.** Verwendung nach Anspruch 3 für die Behandlung von Darmkarzinom.

**12.** Verwendung nach Anspruch 3 oder 6 für die Behandlung von Lungenkrebs.

**13.** Verwendung nach Anspruch 5 für die Behandlung von Brustkrebs.

**14.** Verwendung nach Anspruch 8 für die Behandlung von Nierenklebs.

**15.** Verwendung nach einem der vorstehenden Ansprüche, wobei ET-743 und der andere Wirkstoff als ein einziges Medikament bereitgestellt werden.

**16.** Verwendung nach einem der Ansprüche 1 bis 14, wobei ET-743 und der andere Wirkstoff als getrennte Medikamente bereitgestellt werden.

**17.** Verwendung nach Anspruch 16, wobei das getrennte Medikament, das ET-743 enthält, für die Verabreichung gleichzeitig mit dem anderen Medikament vorgesehen ist, das den anderen Wirkstoff enthält.

**18.** Verwendung nach Anspruch 16, wobei das getrennte Medikament, das ET-743 enthält, für die Verabreichung zu einem anderen Zeitpunkt als das Medikament vorgesehen ist, das den anderen Wirkstoff enthält.

**19.** Verwendung nach einem der vorstehenden Ansprüche, wobei der andere Wirkstoff oder die Kombination durch Liposom- oder Nanokugelkapselung abgegeben wird.

**20.** Verwendung nach einem der vorstehenden Ansprüche, wobei die Kombinationstherapie ferner Dexamethason verwendet.

**21.** Synergistische Kombination von ET-743 und einem anderen Wirkstoff, der unter einem Anthracyclin-Wirkstoff, einem Platin-Wirkstoff, einem Wirkstoff mit Topoisomerase als Target, einem Taxan-Wirkstoff, einem Antimetaboliten-Wirkstoff oder einem antimitotischen Wirkstoff ausgewählt ist, zur Verwendung bei der Behandlung eines Tumors, wie in einem der vorstehenden Ansprüche beschrieben.

**Revendications**

**1.** Utilisation d'une combinaison synergique de ET-743 et d'un autre médicament choisi parmi un médicament d'anthracycline, un médicament de platine, un médicament ciblant une topoisomérase, un médicament de taxane, un médicament anti-métabolite ou un médicament anti-mitotique dans la fabrication d'un médicament pour le traitement d'une tumeur.

**2.** Utilisation selon la revendication 1, dans laquelle la thérapie de combinaison emploie ET-743 et de la doxorubicine.

**3.** Utilisation selon la revendication 1, dans laquelle la thérapie de combinaison emploie ET-743 et du cisplatine.

**4.** Utilisation selon la revendication 1, dans laquelle la thérapie de combinaison emploie ET-743 et du placlitaxel.

**5.** Utilisation selon la revendication 1, dans laquelle la thérapie de combinaison emploie ET-743 et de la gemcitabine.

**6.** Utilisation selon la revendication 1, dans laquelle la thérapie de combinaison emploie ET-743 et SN-38.

**7.** Utilisation selon la revendication 1, dans laquelle la thérapie de combinaison emploie ET-743 et du trimétrexate.

8. Utilisation selon la revendication 1, dans laquelle la thérapie de combinaison emploie ET-743 et de la vinblastine.

9. Utilisation selon l'une quelconque des revendications 2, 3, 4 ou 7 pour le traitement d'un sarcome.

10. Utilisation selon la revendication 3 ou 6 pour le traitement d'un cancer du côlon.

11. Utilisation selon la revendication 3 pour le traitement d'un carcinome intestinal.

12. Utilisation selon la revendication 3 ou 6 pour le traitement d'un cancer du poumon.

13. Utilisation selon la revendication 5 pour le traitement d'un cancer du sein.

14. Utilisation selon la revendication 8 pour le traitement d'un cancer du rein.

15. Utilisation selon l'une quelconque des revendications précédentes, dans laquelle ET-743 et l'autre médicament sont fournis sous forme d'un seul médicament.

16. Utilisation selon l'une quelconque des revendications 1 à 14, dans laquelle ET-743 et l'autre médicament sont fournis sous forme de médicaments séparés.

17. Utilisation selon la revendication 16, dans laquelle le médicament séparé contenant ET-743 est destiné à une administration en même temps que le médicament contenant l'autre médicament.

18. Utilisation selon la revendication 16, dans laquelle le médicament séparé contenant ET-743 est destiné à une administration à un temps différent du médicament contenant l'autre médicament.

19. Utilisation selon l'une quelconque des revendications précédentes, dans laquelle l'autre médicament ou la combinaison est délivré par une encapsulation dans des liposomes ou des nanosphères.

20. Utilisation selon l'une quelconque des revendications précédentes, dans laquelle la thérapie de combinaison emploie en outre de la dexaméthasone.

21. Combinaison synergique de ET-743 et d'un autre médicament choisi parmi un médicament d'anthracycline, un médicament de platine, un médicament ciblant une topoisomérase, un médicament de taxane, un médicament anti-métabolite ou un médicament anti-mitotique pour une utilisation dans le traitement d'une tumeur comme il est décrit dans l'une quelconque des revendications précédentes.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0069441 A

### Non-patent literature cited in the description

- **Minuzzo et al.** *PNAS,* 2000, vol. 97, 6780-84
- **Laska et al.** *Biometrics,* 1994, vol. 50, 834
- **jin et al.** *PNAS,* 2000, vol. 97, 6775
- **Minuzzo et al.** *PNAS,* 2000, vol. 97, 6780
- **Synold et al.** *Nature Med,* 2001, vol. 7, 584
- **Hendriks et al.** *Ann Oncol,* 1999, vol. 10, 1233